Europäisches Patentamt

European Patent Office

Office européen des brevets

(19)

(11) Numéro de publication:

**0 008 248**
**A1**

(12) # DEMANDE DE BREVET EUROPEEN

(21) Numéro de dépôt: 79400455.6

(22) Date de dépôt: 04.07.79

(51) Int. Cl.³: **A 61 F 5/01, A 61 H 1/02**

(30) Priorité: **12.07.78 FR 7820784**

(71) Demandeur: **Société à Responsabilité Limitée dite: SOCIETE DE FABRICATION DE MATERIEL ORTHOPEDIQUE SOFAMOR, 60,62 rue Rothschild, F-62100 Berck-Plage (FR)**

(43) Date de publication de la demande: 20.02.80
Bulletin 80/4

(72) Inventeur: **Cotrel, Yves Paul Charles Alexandre, Villa Kerosen, F-22100 Dinan (FR)**

(84) Etats contractants désignés: **AT BE CH DE GB IT LU NL SE**

(74) Mandataire: **Bonnetat, Christian, Cabinet PROPI Conseils 23 rue de Léningrad, F-75008 Paris (FR)**

(54) **Procédé et dispositif pour la correction de la rotation du rachis dans les courbures scoliotiques.**

(57) Procédé et dispositif pour la correction de la rotation du rachis d'un patient (1) allongé sur un support horizontal (2), ledit rachis ayant subi une déformation en rotation donnant naissance à une gibbosité. Traitement des scolioses.

Ce procédé et dispositif sont caractérisés en ce que l'on dispose sous ladite gibbosité, entre ledit support (2) et le patient (1), un appui souple transversal (17), fixement ancré à son extrémité (5) opposée à ladite gibbosité, et en ce que l'on exerce, sur l'extrémité de l'appui souple (17) proche de la gibbosité et en saillie par rapport au patient (1) une traction dirigée vers le haut.

-1-

Procédé et dispositif pour la correction de la rotation du râchis dans les courbures scoliotiques.

La présente invention concerne un procédé et un dispositif pour la correction de la rotation du râchis dans les courbures scoliotiques.

On sait que certaines maladies, telles que la scoliose structurale, entraînent simultanément une inflexion latérale du râchis et une rotation des vertèbres qui produit une gibbosité thoracique du côté de la convexité de la courbure.

Pour redresser en alignement ledit râchis, on connaît déjà plusieurs procédés et dispositifs d'élongation longitudinale par traction dudit râchis. Cependant, l'expérience prouve que de telles traction et élongation du râchis sont incapables de réduire les déformations en rotation du râchis. Pour ce faire, en complément des élongations et tractions, on enferme le torse du patient dans un corset de plâtre pressant sur la gibbosité de celui-ci pour redresser la rotation dudit râchis. Une telle méthode est particulièrement incommode et désagréable pour le patient. De plus, elle est de longue durée et ses résultats ne sont pas toujours satisfaisants.

La présente invention a pour objet de remédier à ces inconvénients. Elle concerne un procédé et un dispositif pour le redressement de la rotation du râchis, particulièrement efficace et moins incommode et désagréable que la méthode du corset de plâtre. Le procédé et

0008248

le dispositif selon l'invention peuvent être utilisés à tout stade d'une scoliose, mais sont plus particulièrement appropriés à être mis en oeuvre avant que la déformation en rotation entraînant la gibbosité ne soit devenue irréversible et structurelle, c'est-à-dire pendant la croissance du patient.

A cette fin, selon l'invention, le procédé pour la correction de la rotation du râchis d'un patient allongé sur un support horizontal, ledit râchis ayant subi une déformation en rotation donnant naissance à une gibbosité, est remarquable en ce que l'on dispose sous ladite gibbosité entre ledit support et le patient, un appui souple transversal, fixement ancré à son extrémité opposée à ladite gibbosité et en ce que l'on exerce, sur l'extrémité de l'appui souple proche de la gibbosité et en saillie par rapport au patient, une traction dirigée vers le haut.

Ainsi, grâce à une telle traction vers le haut, on applique sur la gibbosité une pression qui exerce ses effets dans le sens d'un redressement en rotation du râchis du patient appuyé contre ledit appui souple par son poids.

Pour faire varier la localisation de cette pression,et donc les effets de redressement engendrés,il suffit de faire varier, à traction égale, l'inclinaison de la ligne d'action de la traction: la pression est d'autant plus externe que l'inclinaison de la ligne d'action de la traction est plus proche de la verticale ou même inclinée vers le côté du patient opposé à la gibbosité (tout en restant dirigée vers le haut) et d'autant plus postérieure que ladite inclinaison se rapproche de l'horizontale, dans un sens opposé au patient.

Dans un premier mode de mise en oeuvre du procédé selon l'invention, qualifié de statique, on ancre fixement à un ancrage élevé, de préférence réglable, l'extrémité de l'appui souple proche de la gibbosité en tendant celui-ci, de sorte que la gibbosité soit soulevée et pressée pour obtenir les effets mentionnés ci-dessus. Un tel mode de mise en oeuvre peut être utilisé la nuit, pendant le sommeil du patient. Il offre l'avantage d'une action continue et égale.

Dans un second mode de mise en oeuvre du procédé selon l'invention, qualifié de dynamique, l'extrémité de l'appui souple proche de la gibbosité est tirée par le patient lui-même, soit avec au moins une main, soit avec au moins un pied. Le patient peut donc alors lui-même régler la traction appliquée.

Bien entendu, le procédé de redressement en rotation selon l'invention peut être associé à une méthode d'élongation, par exemple du type décrit dans le brevet français N° 2 205 815 (72 39480).

Pour la mise en oeuvre du procédé selon l'invention un dispositif comporte un portique fixe dont la partie horizontale est disposée au-dessus dudit support, des moyens d'attache et/ou de renvoi disposés de façon réglable sur ladite partie horizontale du portique, un appui souple transversal passant sous la gibbosité du patient, des moyens d'ancrage de l'extrémité dudit appui souple opposée à la gibbosité et des moyens de liaison de l'extrémité dudit appui souple proche de la gibbosité auxdits moyens d'attache et/ou de renvoi.

Le portique peut être monté sur le support horizontal prévu à cet effet. Il peut également être composé d'éléments assemblables venant enserrer et se solidariser de tout support horizontal non prévu initialement à cet effet. En tout cas, la partie sensiblement horizontale dudit protique comporte au moins une barre longitudinale ou transversale, le long de laquelle peuvent être réglés en position lesdits moyens d'attache et/ou de renvoi.

L'appui souple peut être du type sangle, par exemple recouverte de peau de poulain, pour éviter la détérioration de la peau.

L'extrémité de l'appui souple opposée à la gibbosité est de préférence ancrée de façon réglable longitudinalement, et elle peut être ancrée sur le support horizontal.

Dans le cas du premier mode de mise en oeuvre statique du procédé selon l'invention, les moyens de liaison de l'appui souple sont simplement fixés auxdits moyens d'attache. Dans le cas du second mode de mise en oeuvre dynamique, lesdits organes de liaison de l'appui souple passent sur une poulie de renvoi des moyens de renvoi et à leur extrémité libre ils sont solidaires d'au moins un organe de manoeuvre actionnable par au moins une main ou un pied.

Dans le cas d'association avec une élongation du râchis, le portique peut servir à la fois pour cette élongation et le redressement en rotation. Il en est de même d'au moins certains organes de manoeuvre.

Les figures du dessin annexé feront bien comprendre comment l'invention peut être réalisée.

La figure 1 est une vue de côté schématique illustrant le premier mode de mise en oeuvre du procédé selon l'invention.

La figure 2 est une vue de dessus correspondant à la figure 1.

La figure 3 est une vue de côté schématique illustrant le second mode de mise en oeuvre du procédé selon l'invention.

La figure 4 est une vue de dessus correspondant à la figure 3.

Les figures 5 et 6 illustrent deux autres variantes de mise en oeuvre du procédé selon l'invention, associées à une traction longitudinale du râchis.

La figure 7 montre un mode de réalisation du dispositif selon l'invention.

La figure 8 est une coupe selon la ligne VIII-VIII de la figure 7.

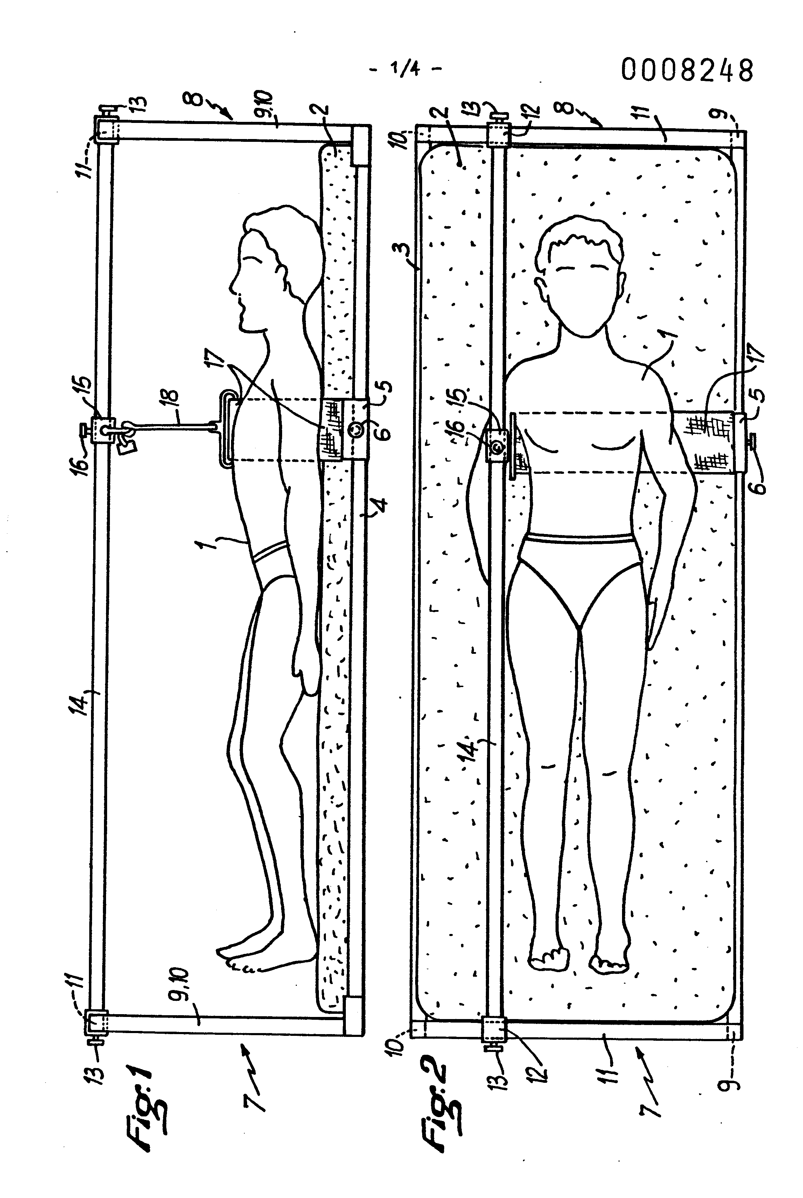

Sur les figures 1 et 2, on a représenté un patient 1 allongé sur un lit 2, dont les pieds n'ont pas été représentés à des fins de clarté. Ce lit 2 comporte des profilés longitudinaux et latéraux 3 et 4 , le long desquels peut coulisser un curseur 5 susceptible d'être fixé en position par une vis de pression 6.

A chaque extrémité du lit 2, deux cadres verticaux 7 et 8 sont montés solidaires de celui-ci. Chaque cadre 7 ou 8 comporte deux montants verticaux 9 et 10 et une barre horizontale 11. Sur chaque barre horizontale 11 est monté un curseur 12 pouvant être fixé en position par une vis de pression 13. Les deux curseurs 12 sont reliés l'un à l'autre par une barre longitudinale 14 sur laquelle peut coulisser un curseur 15, susceptible d'être fixé en position par une vis de pression 16.

Par ailleurs, au curseur 5 est fixé une extrémité d'une sangle transversale 27 passant entre le lit 2 et le patient 1, au niveau de la gibbosité de celui-ci. Le curseur 5 est disposé du côté opposé à ladite gibbosité. L'extrémité de la sangle 17 proche de cette gibbosité est solidaire d'un lien 18, attaché au curseur 15 et tendant la sangle 17.

On voit que par réglage des curseurs 12 et 15, il est possible de donner au lien 18 l'inclinaison désirée par rapport à la verticale et donc d'ajuster le lieu de la pression exercée par la sangle 17 sur la gibbosité du patient 1. De plus par réglage concomitant du curseur 5, on peut ajuster au mieux la position transversale de la sangle 17 par rapport au corps du patient.

Bien entendu, au lieu d'être longitudinale, la barre 14 pourrait être transversale, les cadres 7 et 8 étant alors agencés latéralement au lit 2.

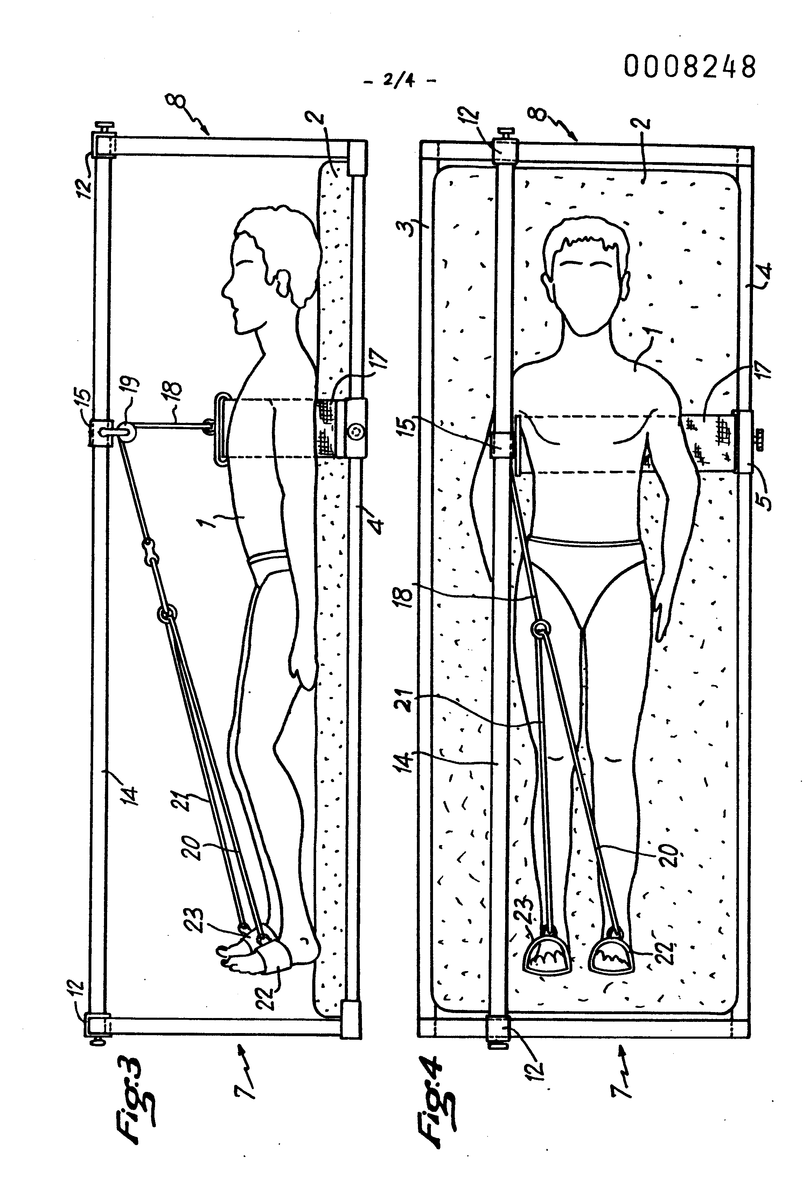

Dans le mode de réalisation montré par les figures 3 et 4, on retrouve le patient 1, le lit 2, les profilés 3 et 4, le curseur 5, les cadres 7 et 8, les curseurs 12, la barre 14 , le curseur 15, la sangle 17 et le lien 18. Toutefois, dans ce cas, le curseur 15 comporte une poulie de renvoi 19 et le lien 18, au lieu d'être fixé audit curseur 15, passe sur ladite poulie 19 et est prolongé par des liens 20 et 21, à chacun desquels est reliée une pédale 22 ou 23 coopérant avec un pied du patient. Dans ce cas, la pression exercée par la sangle 17 par la gibbosité du patient 1 provient de la traction effectuée par les jambes du patient par l'intermédiaire des pédales 22, 23 et des liens 20, 21 et du lien 18. Le patient est donc actif et, psychologiquement, a l'impression de participer efficacement à sa correction.

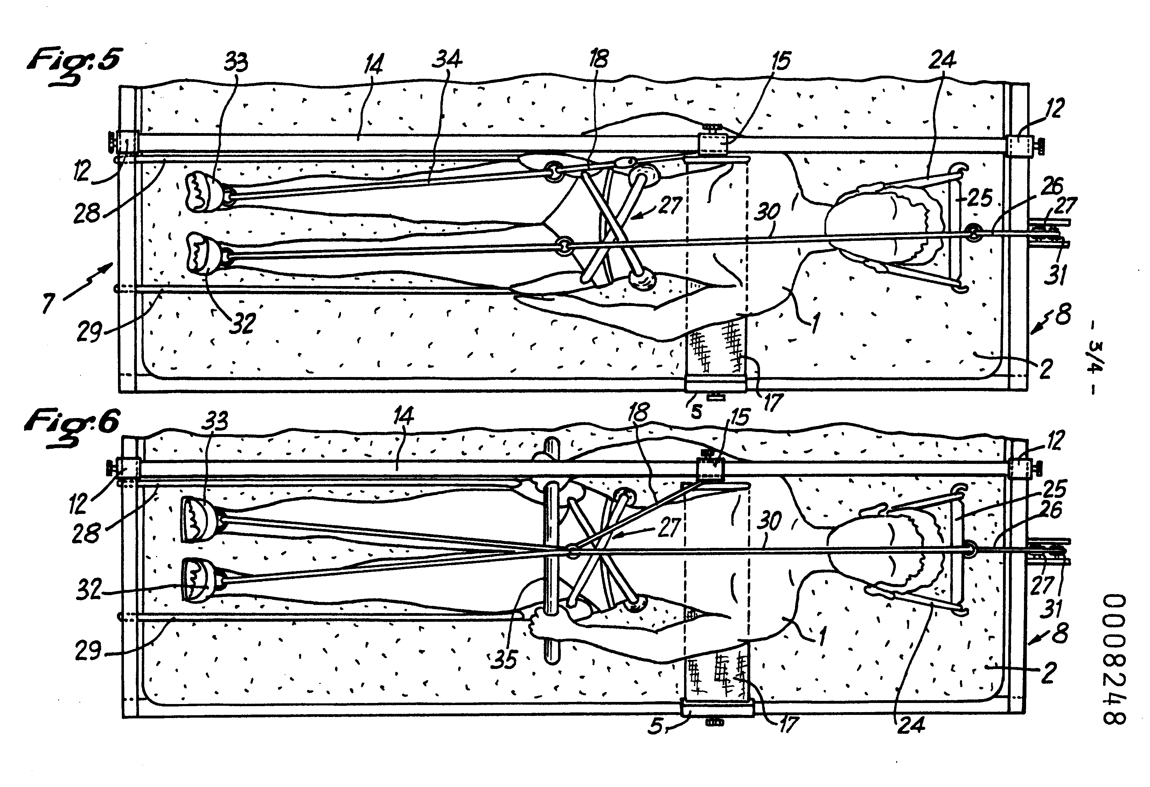

Dans le dispositif de la figure 6, la tête du patient 1 est entourée par un bandeau 24, passant par exemple sous l'occiput et au niveau des tempes et relié aux extrémités d'un palonnier 25. Le palonnier est relié à un contrepoids (non représenté) par l'intermédiaire d'un lien médian 26, passant sur une poulie 27 solidaire du cadre 8.

Du côté opposé, la taille du patient 1 est emprisonnée dans un dispositif de prise pelvienne 27, relié au cadre 7 par des liens 28 et 29. Ainsi, le patient 1 est soumis à une traction longitudinale continue par l'action dudit contrepoids.

A cette traction longitudinale continue, est superposée une traction longitudinale volontaire, car le palonnier 25 est de plus relié, par un lien 30 passant sur une autre poulie 31 solidaire du cadre 8, à une pédale 32 actionnée par le pied gauche du patient 1. Le pied droit dudit patient actionne une pédale 33, reliée par un lien 34, au lien 18 et à la sangle 17. Le dispositif de la figure 5 permet donc simultanément la traction longitudinale continue du rachis, la traction longitudinale volontaire et le redressement en rotation. Toutefois, la traction longitudinale volontaire et le redressement en rotation sont dissociés. Dans le dispositif de la figure 6, ces deux opérations sont non seulement simultanées, mais encore associées. Pour cela, les liens 18 et 30 sont reliés en commun aux pédales 31 et 33, ainsi qu'à une barre de manoeuvre à main 35. Ainsi, en tirant sur les pédales 32 et 33 et/ou sur la barre 35, le patient 1 réalise à la fois l'élongation volontaire de son rachis et le redressement en rotation de celui-ci. Le raccourcissement de l'un ou l'autre des liens permet d'augmenter à volonté la force d'élongation ou de dérotation. Bien entendu, la traction continue par contrepoids peut être maintenue.

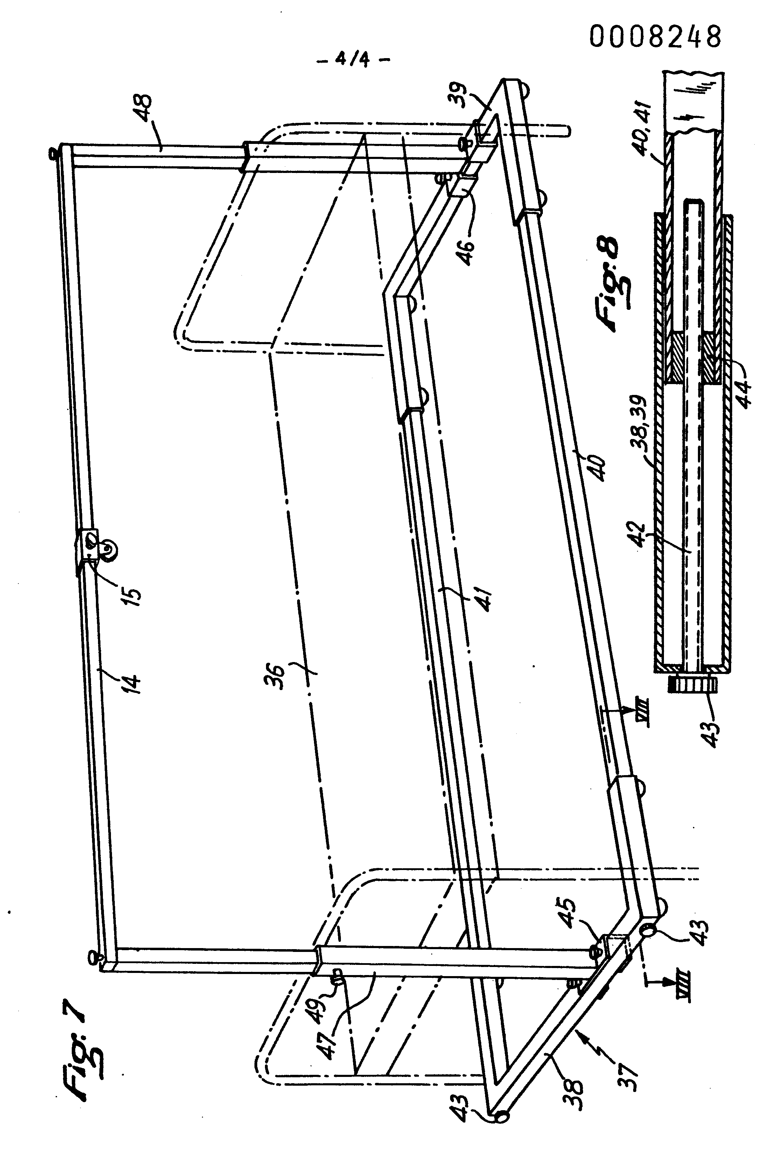

Dans les modes de réalisation décrits sur les figures 1 à 6, le portique 7, 8, 14 était solidaire du lit 2, qui était prévu spécialement pour la mise en oeuvre du procédé selon l'invention. Les figures 7 et 8 illustrent un portique permettant de mettre en oeuvre le procédé de l'invention avec un lit 36 de type quelconque. Ainsi, ce procédé peut être appliqué aux malades chez eux, alors qu'ils sont allongés dans leur lit, ce qui évite l'achat d'un lit spécial ou le dépaysement du traitement dans un lit autre que le sien.

Le portique selon l'invention,montré par la figure 7, comporte un cadre de piètement 37 formé par deux pièces tubulaires d'extrémité 38 et 39, de forme générale en U. Les branches des U 38 et 39 en regard sont reliées par des barres longitudinales 40 et 41, dont les extrémités pénètrent dans lesdites branches. Des vis 42, commandées par des boutons extérieurs 43 et tournant librement par rapport aux branches des U 38 et 39 , coopèrent avec des écrous 44 solidaires des extrémités des barres longitudinales 40 et 41. Ainsi, on peut solidariser lesdites barres des U 38 et 39 et régler la longueur du cadre 37 pour qu'il vienne enserrer en les pressant les pieds du lit 36, de façon à être solidarisé de celui-ci. De plus, on pourrait prévoir les pièces tubulaires 38 et 39 réglables également en largeur, pour s'adapter aux largeurs de différents lits.

Sur les parties médianes des pièces 38 et 39 reliant les branches de celles-ci, sont montés coulissant de façon à pouvoir être fixés en position, des curseurs 45 et 46 lesquels sont solidarisés de montants 47, 48, de préférence télescopiques et fixables en longueur grâce à des vis 49. Sur les montants 47 et 48, est fixée une barre longitudinale 14, pourvue d'un curseur 15.

Grâce à la mise en oeuvre du procédé et du dispositif selon l'invention, la prévention de l'évolution scoliotique et la correction des déformations vertébrales et thoraciques chez l'enfant en croissance sont possibles.

La combinaison des deux forces correctrices d'élongation et de dérotation, mises en oeuvre par le patient lui-même, permet en effet de s'opposer aux deux forces déformantes intervenant simultanément dans le mécanisme d'évolution des scolioses structurales : l'inflexion rachidienne et la rotation vertébrale et thoracique.

R E V E N D I C A T I O N S

1.- Procédé pour la correction de la rotation du râchis d'un patient allongé sur un support horizontal (2), ledit râchis ayant subi une déformation en rotation donnant naissance à une gibbosité, caractérisé en ce que l'on dispose sous ladite gibbosité, entre ledit support et le patient, un appui souple transversal (17), fixement ancré à son extrémité (5) opposée à ladite gibbosité et, en ce que l'on exerce, sur l'extrémité de l'appui souple (17) proche de la gibbosité et en saillie par rapport au patient (1), une traction dirigée vers le haut.

2.- Procédé selon la revendication 1, caractérisé en ce que l'on fait varier l'inclinaison de la ligne d'action (18) de la traction vers le haut.

3.- Procédé selon la revendication 1, caractérisé en ce qu'on ancre fixement en hauteur l'extrémité de l'appui souple proche (17) de la gibbosité en tendant celui-ci.

4.- Procédé selon la revendication 1, caractérisé en ce que l'extrémité de l'appui souple (17) proche de la gibbosité est tirée par le patient lui-même, soit avec au moins une main, soit avec au moins un pied.

5.- Procédé selon la revendication 1, caractérisé en ce qu'il est associé à un processus de traction longitudinal du râchis.

6.- Dispositif pour la mise en oeuvre du procédé selon l'une des revendications 1 à 5, caractérisé en ce qu'il comporte un portique fixe (7, 8, 14) dont la partie horizontale (14) est disposée au-dessus dudit support (2), des moyens d'attache et/ou de renvoi (15,19) disposés de façon réglable sur ladite partie horizontale (14) du portique, un appui souple transversal (17) passant sous la gibbosité du patient, des moyens d'ancrage (5,6) de l'extrémité dudit appui souple (17) opposée à la gibbosité et des moyens de liaison (18) de l'extrémité dudit appui souple (17) proche de la gibbosité auxdits moyens d'attache et/ou de renvoi.

7.- Dispositif selon la revendication 6, caractérisé en ce qu'il comporte au moins une barre (14) le long de laquelle peuvent être réglés en position lesdits moyens d'attache et/ou de renvoi (15,19).

8.- Dispositif selon la revendication 6, caractérisé en ce que lesdits moyens d'attache et/ou de renvoi (15,19) comportent au moins une poulie de renvoi (19) sur laquelle passent lesdits moyens de liaison (18), l'extrémité libre de ceux-ci étant solidaire d'au moins un organe de manoeuvre (22, 23, 32, 33) actionnable par au moins une main ou un pied.

9.- Dispositif selon la revendication 6, caractérisé en ce que ledit portique est solidaire d'un support (37) qui est prévu à cet effet.

10.- Dispositif selon la revendication 6, caractérisé en ce que ledit portique (7,8,14) est composé d'éléments assemblables venant enserrer et se solidariser de tout support horizontal non prévu à cet effet.

Fig. 1

Fig. 2

- 1/4 -

0008248

0008248

Fig.3

Fig.4

Fig. 5

Fig. 6

0008248

Fig. 7

14    15    48

36

47  49

43

38

45

37

43

46    39

40

41

- 4/4 -

0008248

Fig. 8

42    38,39    40,41

43

44

Office européen
des brevets

**RAPPORT DE RECHERCHE EUROPEENNE**

0008248
Numéro de la demande

EP 79 400 455.6

## DOCUMENTS CONSIDERES COMME PERTINENTS

| Catégorie | Citation du document avec indication, en cas de besoin, des parties pertinentes | Revendication concernée |
|---|---|---|
| | AT - B - 7 947 (E. REPKE et al.) <br> * page 2, lignes 21 et 22; fig. 3, position h, o * <br> -- | 1-3,6 |
| | DE - C - 152 892 (A. KAISIN) <br> * revendication 1; page 1; page 2, lignes 60 à 64, 87 à 93; page 3, lignes 35 à 98; fig., positions 31, 33, 42 * <br> -- | 1,2, 5,6 |
| | FR - A - 829 380 (F. SALVATI) <br> * page 1; fig. 1 * <br> -- | 1,6-8 |
| | FR - A - 1 428 272 (L.L. ROGERS III) <br> * fig. 1 * <br> -- | 7,10 |
| D,A | FR - A - 2 205 815 (Y.P.C.A. COTREL) <br> * fig. 1, 2 * <br> & DE - A - 2 355 848 <br> ---- | |

**CLASSEMENT DE LA DEMANDE (Int. Cl.3)**

A 61 F 5/01
A 61 H 1/02

**DOMAINES TECHNIQUES RECHERCHES (Int. Cl.3)**

A 61 F 5/00
A 61 G 7/06
A 61 H 1/00
A 63 B 23/02

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

X Le présent rapport de recherche a été établi pour toutes les revendications

| Lieu de la recherche | Date d'achèvement de la recherche | Examinateur |
|---|---|---|
| Berlin | 07-11-1979 | DROPMANN |

OEB Form 1503.1 06.78